# EUROPEAN PATENT APPLICATION

(11) **EP 4 733 416 A1**
(43) Date of publication of application: **29.04.2026**
(21) Application number: 25210581.2
(22) Date of filing: 22.10.2025
(51) Int. Cl.: C12Q 1/6886

(54) **SET OF PRIMERS FOR DETECTING ONCOGENIC MUTATIONS IN DNA, METHOD FOR DETECTING ONCOGENIC MUTATIONS IN DNA, AND ITS USE IN ASSESSING CANCER ORIGIN OF CELLS**

(30) Priority: 22.10.2024 PL 45009224
(71) Applicant: Gdanski Uniwersytet Medyczny, 80-210 Gdansk (PL); Uniwersytet Gdanski, 80-309 Gdansk (PL)
(72) Inventor: STANKIEWICZ, Agnieszka, 81-053 Gdynia (PL); MARKIEWICZ, Aleksandra, 80-858 Gdansk (PL); TOPA, Justyna, 80-180 Gdansk (PL); RYBICKA-MISIEJKO, Magda, 80-175 Gdansk (PL)
(74) Representative: JD&P Patent Attorneys Joanna Dargiewicz & Partners

(57) **Abstract**

The subject of the present invention is a set of primers for detecting oncogenic mutations, at the level of single circulating cancer cells, in the *TP53, PIK3CA, ERBB2, ESR1, CDH1* and *AKT1* genes, a method for detecting oncogenic mutations at the level of single cancer cells in the *TP53, PIK3CA, ERBB2, ESR1, CDH1* and *AKT1* genes using mass spectrometry technology, comprising the steps of amplifying genome fragments containing selected oncogenic mutations, and the use of the present method for assessing the neoplastic origin of cells, analyzing the heterogeneity of a cancer disease and the presence of metastatic cells in the body.

## Description

The present invention relates to a set of primers for detecting oncogenic mutations in DNA, in particular at the level of single cells using mass spectrometry technology, and a method for detecting oncogenic mutations in DNA using primers from the set, as well as the use of the method for assessing the cancerous origin of cells and analyzing the heterogeneity of a cancer disease.

Detection of circulating tumor cells (CTCs) in cancer patients is an innovative approach to assessing tumor aggressiveness or monitoring treatment effectiveness.

Methods for identifying oncogenic mutations in potential CTCs (including methods using mass spectrometry) have been described in the literature. In the publication by Khoo et al., 2014, concerning the use of mass spectrometry to analyze mutations in CTCs, studies were performed on the entire lymphocyte fraction that may contain potential CTCs. Therefore, these were not analyses of individual CTCs, but rather fractions possibly containing CTCs (rare cells, occurring in the blood at low frequency) contaminated with blood cells.

In a conference report by Stilwell et al. (2017), mass spectrometry was used to detect oncogenic mutations. The DNA of single cells was amplified using the Picoplex method, or alternatively, mutations were searched for without amplifying the entire cell genome. This, however, resulted in lower sensitivity (fewer mutations detected) and the inability to analyze mutations in other regions of the genome. Without whole-genome amplification, the genetic material from a single cell is entirely used to analyze a given mutation.

In the publication by Vishnoi et al. (2015), the use of mass spectrometry to analyze mutations in single-cell genomes amplified using Ampli1^{™} WGA technology was described. Of the 200 genes analyzed, mutations were confirmed in 4 genes whose status in the patient's healthy cells was not shown, and information was not provided for which of the 200 genes it was possible to obtain results at the level of single cells preamplified using Ampli1^{™} WGA technology.

In the publication by Peeters et al. (2013), single tumor cells from a breast cancer cell line preamplified with the Ampli1^{™} WGA technology were used. 10 mutations in 6 genes (*ARID1A*, *BRAF, KRAS, NF2, PIK3CA, P53*) were then tested using the iPLEX kit. For 5 mutations, no (reliable) mutation determination result could be obtained.

In the publication by Georges et al. (2019), DNA from single CTCs was amplified using the Ampli1^{™} WGA method, and the product was then screened for 61 mutations using a mass spectrometry-based method-UltraSEEK^{™} Melanoma Panel v1.0 (Agena Bioscience, Hamburg, Germany). The UltraSEEK^{™} method amplified only the variant with the mutation. This method requires biotinylation of the primers and their purity to HPLC standards.

A problem with detecting CTCs is the uncertainty regarding their neoplastic origin. Current detection methods rely on markers and features that cannot fully confirm the neoplastic origin of potential CTCs. As a result, not all cells considered CTCs are actually cancer cells, which increases the number of false positives. A possible solution is to subject CTCs to further molecular analysis (e.g., whole-genome/exome sequencing), which, however, is time-consuming and expensive, and is hampered by administrative implications (such as the need to protect patient genomic data during whole-genome sequencing). A possible solution to this problem is the use of screening methods that test for the neoplastic origin of CTCs, based on the identification of mutations at specific genomic locations. Because cancer development is associated with the acquisition of genomic mutations (so-called somatic mutations) by cancer cells, the presence of these changes can be a criterion confirming the cells' neoplastic origin.

The aim of the invention is to solve the problem of confirming the cancer origin of rare cells such as circulating tumor cells (CTCs). The possibility of further molecular analysis and examination of genetic changes in these cells (e.g., oncogenic mutations) allows us to determine whether the cells are of cancerous origin and, therefore, to determine whether the identified individual cells are indeed circulating cancer cells and whether they may negatively impact cancer progression. Furthermore, the method according to the invention allows us to obtain a result of the mutation status of the tested cell within a short time (approximately 8 hours).

The method of the invention enables the detection of oncogenic mutations using mass spectrometry (using a mass spectrometry analyzer) in DNA isolated from cells and tissues, as well as in DNA from a single cell (e.g., a circulating tumor cell - CTC). A single cell contains very small amount of DNA, therefore its genome must be amplified to allow for broader analysis. There are several methods for whole genome amplification (WGA) of a single cell. One of the most commonly used is the Ampli1^{™} WGA method (Menarini Silicon Biosystems), which is characterized by high genome coverage and low allelic dropout rate. As a result of using Ampli1^{™} WGA, the genome of a single cell is "cut" into restriction fragments of various lengths (due to the action of the restriction enzyme Msel). Enzyme digestion is an extremely important process that must be taken into account when planning further DNA analyses after Ampli1^{™} WGA, because after digestion with the Msel restriction enzyme, not all changes in DNA can be detected due to DNA strand cleavage (the template sequence for primer design is not arbitrary, as is the case when designing primers for mutation detection in material not subjected to the Ampli1^{™} WGA procedure). The method for analyzing mutations in single cells according to the invention has also been adapted to material digested with the Msel restriction enzyme. The primers used for mutation detection using mass spectrometry on the mass spectrometry analyzer were designed and selected to encompass the site of the oncogenic mutation, but at the same time "fit" within the optimal lengths of Msel restriction fragments. However, the method can be used to analyze DNA material amplified by another method (e.g. Picoplex WGA, REPLI-g, MALBAC).

### The summary of the invention

The present invention relates to a set of primers for detecting oncogenic mutations at the level of single circulating tumor cells, in the *TP53, PIK3CA, ERBB2*, *ESR1*, *CDH1* and *AKT1* genes, characterized in that the forward primer for amplifying the polymorphism in the *TP53* gene is selected from SEQ ID NO.1 to SEQ ID NO.7, the reverse primer is selected from the group of SEQ ID NO.24 to SEQ ID NO.30, the forward primer for amplifying the polymorphism in the *PIK3CA* gene is selected from the group of SEQ ID NO.8 to SEQ ID NO.14, the reverse primer is selected from the group of SEQ ID NO.31 to SEQ ID NO.37, the forward primer for amplifying the polymorphism in the *ERBB2* gene is selected from SEQ ID NO.15 to SEQ ID NO. 17, the reverse primer is selected from the group of SEQ ID NO.38 to SEQ ID NO.40, the forward primer for amplifying the polymorphism in the *ESR1* gene is selected from the group of SEQ ID NO.18 to SEQ ID NO.21, the reverse primer is selected from the group of SEQ ID NO.41 to SEQ ID NO.44, the forward primer for amplifying the polymorphism in the *CDH1* gene is SEQ ID NO.22, the reverse primer is SEQ ID NO.45, the forward primer for amplifying the polymorphism in the *AKT1* gene is SEQ ID NO.23, the reverse primer is SEQ ID NO.46, wherein the kit comprises a differentiating primer which for the *TP53* gene is selected from the group of SEQ ID NO.47 to SEQ ID NO.53, for the *PIK3CA* gene is selected from the group of SEQ ID NO.54 to SEQ ID NO.60, for the *ERBB2* gene is selected from the group of SEQ ID NO.61 to SEQ ID NO.63, for the *ESR1* gene is selected from the group SEQ ID NO.64 to SEQ ID NO.67, for the *CDH1* gene is SEQ ID NO.68, for the *AKT1* gene is SEQ ID NO.69.

Preferably, the set of primers is characterized in that the forward primer has the sequence SEQ ID NO.1, the reverse primer has the sequence SEQ ID NO.24 and the differentiating primer has the sequence SEQ ID NO.47.

Preferably, when the forward primer has the sequence SEQ ID NO.2, the reverse primer has the sequence SEQ ID NO.25 and the differentiating primer has the sequence SEQ ID NO.48.

Preferably, when the forward primer has the sequence SEQ ID NO.3, the reverse primer has the sequence SEQ ID NO.26 and the differentiating primer has the sequence SEQ ID NO.49.

Preferably, when the forward primer has the sequence SEQ ID NO.4, the reverse primer has the sequence SEQ ID NO.27 and the differentiating primer has the sequence SEQ ID NO.50.

Preferably, when the forward primer has the sequence SEQ ID NO.5, the reverse primer has the sequence SEQ ID NO.28 and the differentiating primer has the sequence SEQ ID NO.51.

Preferably, when the forward primer has the sequence SEQ ID NO.6, the reverse primer has the sequence SEQ ID NO.29 and the differentiating primer has the sequence SEQ ID NO.52.

Preferably, when the forward primer has the sequence SEQ ID NO.7, the reverse primer has the sequence SEQ ID NO.30 and the differentiating primer has the sequence SEQ ID NO.53.

Preferably, when the forward primer has the sequence SEQ ID NO.8, the reverse primer has the sequence SEQ ID NO.31 and the differentiating primer has the sequence SEQ ID NO.54.

Preferably, when the forward primer has the sequence SEQ ID NO.9, the reverse primer has the sequence SEQ ID NO.32 and the differentiating primer has the sequence SEQ ID NO.55.

Preferably, when the forward primer has the sequence SEQ ID NO.10, the reverse primer has the sequence SEQ ID NO.33 and the differentiating primer has the sequence SEQ ID NO.5 6.

Preferably, when the forward primer has the sequence SEQ ID NO.11, the reverse primer has the sequence SEQ ID NO.34 and the differentiating primer has the sequence SEQ ID NO.57.

Preferably, when the forward primer has the sequence SEQ ID NO.12, the reverse primer has the sequence SEQ ID NO.35 and the differentiating primer has the sequence SEQ ID NO.58.

Preferably, when the forward primer has the sequence SEQ ID NO.13, the reverse primer has the sequence SEQ ID NO.36 and the differentiating primer has the sequence SEQ ID NO.59.

Preferably, when the forward primer has the sequence SEQ ID NO.14, the reverse primer has the sequence SEQ ID NO.37 and the differentiating primer has the sequence SEQ ID NO.60.

Preferably, when the forward primer has the sequence SEQ ID NO.15, the reverse primer has the sequence SEQ ID NO.38 and the differentiating primer has the sequence SEQ ID NO.61.

Preferably, when the forward primer has the sequence SEQ ID NO.16, the reverse primer has the sequence SEQ ID NO.39 and the differentiating primer has the sequence SEQ ID NO.62.

Preferably, when the forward primer has the sequence SEQ ID NO.17, the reverse primer has the sequence SEQ ID NO.40 and the differentiating primer has the sequence SEQ ID NO.63.

Preferably, when the forward primer has the sequence SEQ ID NO.18, the reverse primer has the sequence SEQ ID NO.41 and the differentiating primer has the sequence SEQ ID NO.64.

Preferably, when the forward primer has the sequence SEQ ID NO.19, the reverse primer has the sequence SEQ ID NO.42 and the differentiating primer has the sequence SEQ ID NO.65.

Preferably, when the forward primer has the sequence SEQ ID NO.20, the reverse primer has the sequence SEQ ID NO.43 and the differentiating primer has the sequence SEQ ID NO.66.

Preferably, when the forward primer has the sequence SEQ ID NO.21, the reverse primer has the sequence SEQ ID NO.44 and the differentiating primer has the sequence SEQ ID NO.67.

Preferably, when the forward primer has the sequence SEQ ID NO.22, the reverse primer has the sequence SEQ ID NO.45 and the differentiating primer has the sequence SEQ ID NO.68.

Preferably, when the forward primer has the sequence SEQ ID NO.23, the reverse primer has the sequence SEQ ID NO.46 and the differentiating primer has the sequence SEQ ID NO.69.

Even more preferably, the constant sequence ACGTTGGATG is present in the sequence of each primer, necessary for the detection of the amplified DNA in a mass spectrometer.

Another subject of the invention is a method for detecting oncogenic mutations at the level of single cancer cells in the *TP53, PIK3CA, ERBB2, ESR1, CDH1* and *AKT1* genes using mass spectrometry technology, comprising the steps of cell lysis and DNA digestion with the Msel restriction enzyme, DNA amplification from a single cell using the commercial Ampli1^{™} WGA kit and amplification of genome fragments containing selected oncogenic mutations, characterized in that genome fragments containing an oncogenic mutation, including sequences between the sites of cleavage with the Msel restriction enzyme in step a) are amplified using a set of primers comprising a forward primer and a reverse primer, in step b) the product obtained in step a) is treated with alkaline phosphatase, in step c) the product from step b) is subjected to iPLEX amplification with a differentiating primer to detect a specific mutation, then in step d) the product from step c) is purified on an ion exchange resin, after which in step e) MALDI-TOF analysis of the iPLEX reaction products is performed in a spectrometer and their mass spectra are compared, and in step f) the genotype of the sample is assessed on the basis of the detected alleles and the results are interpreted.

Preferably in the method the forward primer for amplifying the polymorphism in the *TP53* gene in step a) is selected from SEQ ID NO.1 to SEQ ID NO.7 and the reverse primer is selected from SEQ ID NO.24 to SEQ ID NO.30.

Preferably in the method the forward primer for amplifying the polymorphism in the *PIK3CA* gene in step a) is selected from SEQ ID NO.8 to SEQ ID NO.14 and the reverse primer is selected from SEQ ID NO.31 to SEQ ID NO.37.

Preferably, in the method, the forward primer for amplifying the polymorphism in the *ERBB2* gene in step a) is selected from SEQ ID NO. 15 to SEQ ID NO. 17, and the reverse primer is selected from SEQ ID NO. 38 to SEQ ID NO. 40.

Preferably in the method the forward primer for amplifying the polymorphism in the *ESR1* gene in step a) is selected from SEQ ID NO.18 to SEQ ID NO.21 and the reverse primer is selected from SEQ ID NO.41 to SEQ ID NO.44.

Preferably in the method the forward primer for amplifying the polymorphism in the *CDH1* gene in step a) is SEQ ID NO.22 and the reverse primer is SEQ ID NO.45.

Preferably, in the method, the forward primer for amplifying the polymorphism in the *AKT1* gene in step a) is SEQ ID NO.23 and the reverse primer is SEQ ID NO.46.

Preferably, in the method, the amplification in step a) takes place in a thermal cycler according to the scheme: 2 minutes at 95°C, then 45 cycles of: 30 seconds at 95°C; 30 seconds at 56°C; 60 seconds at 72°C and includes a final step of 5 minutes at 72°C.

Preferably, in step b) the incubation with alkaline phosphatase is at 36-38 °C for at least 40 minutes.

Preferably, in step b) the incubation with alkaline phosphatase is at 37 °C for 45 minutes.

Preferably, the iPLEX reaction products of step c) are purified in step d) with 15 mg of ion exchange resin by centrifugation for 15 minutes at 3200xg.

Preferably in step e) the products are applied to the analysis plate and subjected to a UV laser pulse with a wavelength of 337 nm exciting the ions, preferably the analysis takes place in a vacuum.

Preferably, the analysis plate used in the method of the invention is a SpectroCHIP.

Another subject of the invention is the use of a method for assessing the cancer origin of cells and to analyze the heterogeneity of cancer and the presence of metastatic cells in the body.

### Brief description of the figures

Fig. 1. Schematic view of the method's steps for analyzing the presence of mutations in DNA from the tested samples.
Fig. 2. Shows the result of analysis of the rs121913343 (c.817C>T) polymorphism in the *TP53* gene in DNA from a single cell with a mutant allele, in which a peak for the detected adenine nucleotide is visible (nucleotide A indicated by an arrow, connecting to T) and a wild-type allele, in which a peak for the detected guanine nucleotide is visible (nucleotide G indicated by an arrow, connecting to C)

### Examples of use

The panel of detected mutations according to the invention enables the detection of 23 mutations in 6 genes: *TP53, PIK3CA, ERBB2, ESR1, CDH1*, and *AKT1* (Table 1), which are among the most frequently mutated in breast cancer. This panel can be expanded to include additional mutations.

**Table 1. Panel of mutations detected using the developed method for identifying mutations in single cells.**

| **Gene** | **Polymorphism number** | **Nucleotide change** |
|---|---|---|
| *TP53* | rs28934576 | c.818G>A |
| | rs121912651 | c.742C>T |
| | rs11540652 | c.743G>A |
| | rs121913343 | c.817C>T |
| | rs397516436 | c.637C>T |
| | rs28934578 | c.524G>A |
| | rs121912666 | c.659A>G/C |
| *PIK3CA* | rs121913279 | c.3140A>G |
| | rs121913273 | c.1624G>A |
| | rs104886003 | c.1633G>C |
| | rs121913284 | c.1035T>A |
| | rs121913277 | c.3145G>C |
| | rs121913274 | c.1634A>C/G |
| | rs121913286 | c.1636C>A/G |
| *ERBB2* | rs1057519816 | c.929C>T/A |
| | rs121913471 | c.2329G>T/C/A |
| | rs121913470 | c.2264T>C/G |
| *ESR1* | rs796065354 | c.908A>G |
| | rs1057519717 | c.1607T>A/C/G |
| | rs2152506534 | c.1609T>A/C/G |
| | rs2152506541 | c.1610A>C/G |
| *CDH1* | rs1962456814 | c.67C>T |
| *AKT1* | rs121434592 | c.49G>A |

To detect the above-mentioned mutations, primers were designed for each polymorphism (Table 2), for 7 polymorphisms in the *TP53* gene, 7 polymorphisms in the *PIK3CA* gene, 3 polymorphisms in the *ERBB2* gene, 4 polymorphisms in the *ESR1* gene, 1 polymorphism in the *CDH1* gene, and 1 polymorphism in the *AKT1* gene. The primers were designed using software dedicated to the mass spectrometry analyzer system (Mass Assay Designer software package, version 4.0). The products of the first reaction, amplified using a pair of primers (Primer 1 and Primer 2 for each polymorphism), were checked using an in-silico PCR analysis tool (UCSC In-Silico PCR) to verify the presence or absence of Msel restriction sites in the amplicon bounded by the sequence of Primers 1 and Primer 2. If restriction sites were present in the amplicon, the primers were redesigned until the desired result was achieved-an amplicon without the Msel restriction site. Primer systems detecting a given polymorphism were combined to minimize the number of reactions (so-called multiplex reaction), but to ensure that the polymorphism was distinguishable by a mass spectrometer (the spectrometer has a resolution of 16 Da). After the selected primers were synthesized, their concentrations were optimized to ensure proper reaction performance. To increase the sensitivity of the method, 3 µl of the WGA product was dried before starting the reaction to obtain a higher DNA concentration (minimum 50 ng), and the final PCR products (9 µl) were diluted in 35 µl of water.

**Table 2. Identification numbers of polymorphisms and primer sequences used to detect them in the method according to the invention. The bold sequence ACGTTGGATG in the sequence of the forward primer and the reverse primer is a constant element necessary for detection of the product in the mass spectrometer.**

| **Gene** | **Polymorphism number** | **Forward primer sequence (5' →3')** | **Reverse primer sequence (5' →3')** | **Differentiation primer sequence (5' →3')** |
|---|---|---|---|---|
| | rs28934576 | SEQ ID NO.1 ACGTTGGATG AGATTCTCTTCCTCT GTGCG | SEQ ID NO.24 ACGTTGGATG AGTGGTAATCTACTG GGACG | SEQ ID NO.47 GGACAGCTTTGAG GTGC |
| *TP53* | rs121912651 | SEQ ID NO.2 ACGTTGGATG CCACCATCCACTAC AACTAC | SEQ ID NO.25 ACGTTGGATG TCCAGTGTGATGAT GGTGAG | SEQ ID NO.48 TTGAGGATGGGCC TCC |
| | rs11540652 | SEQ ID NO.3 ACGTTGGATG TCCAGTGTGATGAT GGTGAG | SEQ ID NO.26 ACGTTGGATG CCACCATCCACTAC AACTAC | SEQ ID NO.49 TGGGCGGCATGAA CC |
| | rs121913343 | SEQ ID NO.4 ACGTTGGATG AGATTCTCTTCCTCT GTGCG | SEQ ID NO.27 ACGTTGGATG AGTGGTAATCTACTG GGACG | SEQ ID NO.50 CGGAACAGCTTTG AGGTG |
| | rs397516436 | SEQ ID NO.5 ACGTTGGATG AGTTGCAAACCAGA CCTCAG | SEQ ID NO.28 ACGTTGGATG GTGTGGAGTATTTG GATGAC | SEQ ID NO.51 ATGACAGAAACAC TTTT |
| | rs28934578 | SEQ ID NO.6 ACGTTGGATG TGCTCACCATCGCT ATCTG | SEQ ID NO.29 ACGTTGGATG CCATCTACAAGCAG TCACAG | SEQ ID NO.52 CGGAGGTTGTGAG GC |
| | rs121912666 | SEQ ID NO.7 ACGTTGGATG AGTTGCAAACCAGA CCTCAG | SEQ ID NO.30 ACGTTGGATG GTGTGGAGTATTTG GATGAC | SEQ ID NO.53 ATAGTGTGGTGGT GCCCT |
| *PIK3CA* | rs121913279 | SEQ ID NO.8 ACGTTGGATG AACTGAGCAAGAGG CTTTGG | SEQ ID NO.31 ACGTTGGATG TCCATTTTTGTTGTC CAGCC | SEQ ID NO.54 GTCCAGCCACCAT GA |
| | rs121913273 | SEQ ID NO.9 ACGTTGGATG TAGCACTTACCTGTG ACTCC | SEQ ID NO.32 ACGTTGGATG GCAATTTCTACACGA GATCC | SEQ ID NO.55 CACGAGATCCTCT CTCT |
| | rs104886003 | SEQ ID NO.10 ACGTTGGATG TAGCACTTACCTGTG ACTCC | SEQ ID NO.33 ACGTTGGAT GTACACGAGATCCT CTCTCTG | SEQ ID NO.56 CCTCTCTCTGAAA TCACT |
| | rs121913284 | SEQ ID NO.11 ACGTTGGATG GGGTTATAAATAGTG CACTC | SEQ ID NO.34 ACGTTGGATG CATCAGCATTTGACT TTACC | SEQ ID NO.57 CCTTATCAATGTC TCGAATATTTAC |
| | rs121913277 | SEQ ID NO.12 ACGTTGGATG AACTGAGCAAGAGG CTTTGG | SEQ ID NO.35 ACGTTGGATG TTGTGTGGAAGATC CAATC | SEQ ID NO.58 ATTTTTGTTGTCCA GCCAC |
| | rs121913274 | SEQ ID NO.13 ACGTTGGATG TAGCACTTACCTGTG ACTCC | SEQ ID NO.36 ACGTTGGATG TACACGAGATCCTC TCTCTG | SEQ ID NO.59 TCTCTCTGAAATC ACTG |
| | rs121913286 | SEQ ID NO.14 ACGTTGGATG TACACGAGATCCTC TCTCTG | SEQ ID NO.37 ACGTTGGATG TAGCACTTACCTGTG ACTCC | SEQ ID NO.60 AGAAAATCTTTCT CCT |
| *ERBB2* | rs1057519816 | SEQ ID NO.15 ACGTTGGATG TCCTGATCTCCTTAG ACAAC | SEQ ID NO.38 ACGTTGGATG TCACCTCTTGGTTGT GCAG | SEQ ID NO.61 GGGCAGACGAGG GTGCAG |
| | rs12191347 1 | SEQ ID NO.16 ACGTTGGATG CGTGGATGTCAGGC AGATG | SEQ ID NO.39 ACGTTGGATG CCAGGAAGCATACG TGATG | SEQ ID NO.62 AAGCATACGTGAT GGCTGGT |
| | rs121913470 | SEQ ID NO.17 ACGTTGGATG GGGCTTACGTCTAA GATTTC | SEQ ID NO.40 ACGTTGGATG GAAAATTCCAGTGG CCATC | SEQ ID NO.63 GTGGCCATCAAAG TGT |
| *ESR1* | rs796065354 | SEQ ID NO.18 ACGTTGGATG AAGCCCGCTCATGA TCAAAC | SEQ ID NO.41 ACGTTGGATG AACAAGGCACTGAC CATCTG | SEQ ID NO.64 GGACAAGGCCAG GCTGTTC |
| | rs1057519717 | SEQ ID NO.19 ACGTTGGATG CAGCATGAAGTGCA AGAACG | SEQ ID NO.42 ACGTTGGATG GCTAGTGGGCGCAT GTAGG | SEQ ID NO.65 CATCTCCAGCAGC AGGTCATAG |
| | rs2152506534 | SEQ ID NO.20 ACGTTGGATG CAGCATGAAGTGCA AGAACG | SEQ ID NO.43 ACGTTGGATG GCTAGTGGGCGCAT GTAGG | SEQ ID NO.66 CCAGCAGCAGGTC AT |
| | rs2152506541 - | SEQ ID NO.21 ACGTTGGATG CAGCATGAAGTGCA AGAACG | SEQ ID NO.44 ACGTTGGATG GCTAGTGGGCGCAT GTAGG | SEQ ID NO.67 ATCTCCAGCAGCA GGTCA |
| *CDH1* | rs1962456814 - | SEQ ID NO.22 ACGTTGGATG CCGGTTCCATCTAC CTTTC | SEQ ID NO.45 ACGTTGGATG TGTAGCTCTCGGCG TCAAAG | SEQ ID NO.68 GGGTGGCAGGGC TCCGGCTCCT |
| *AKT1* | rs121434592 | SEQ ID NO.23 ACGTTGGATG TCTGACGGGTAGAG TGTGC | SEQ ID NO.46 ACGTTGGATG TTCTTGAGGAGGAA GTAGCG | SEQ ID NO.69 GTGGCCGCCAGG TCTTGATGTACT |

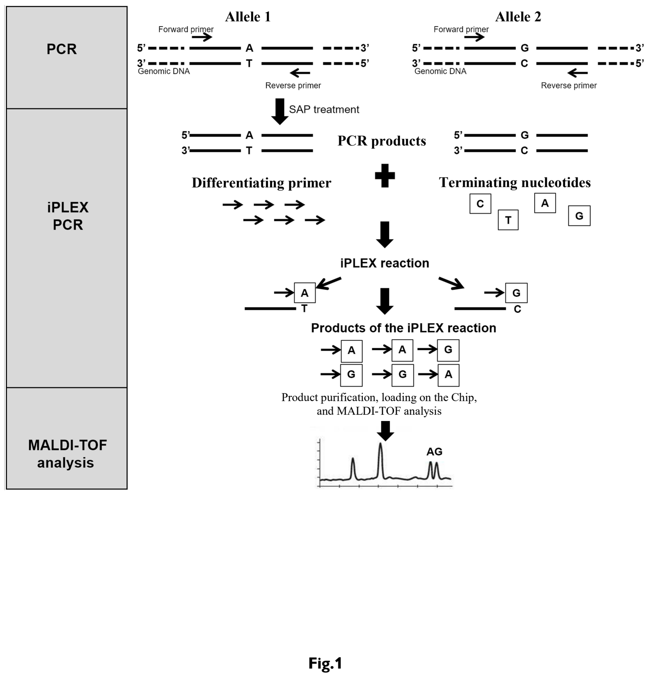

The steps of laboratory analysis of polymorphisms in samples are shown in Fig. 1 and the compositions of reaction mixtures are shown in Table 3).

**Table 3. Composition of reaction mixtures based on the example of detection of the rs28934576 polymorphism in the TP53 gene. In the case of other polymorphisms, only the primer sequences given in Table 2 are changed.**

| **First stage of reaction:** | | |
|---|---|---|
| | **Stock concentration** | **Volume [µl]** |
| Forward primer, SEQ ID NO.1 | 500 nM | 0.5 |
| Reverse primer, SEQ ID NO.24 | 500 nM | 0.5 |
| MgCl₂ | 25 mM | 0.4 |
| PCR buffer | 10x | 0.5 |
| dNTPs | 25 mM | 0.1 |
| Taq polymerase | 5 U/µl | 0.2 |
| DNA | variable | variable |
| H₂O | | 2.8 µl or make up to 5 µl |
| **Total reaction volume** | **-** | **5** |

### Second stage of reaction:

| | **Stock concentration** | **Volume [µl]** |
|---|---|---|
| Mixture from stage 1 | | 5 |
| SAP | 1.7 U/µl | 0.3 |
| SAP buffer | 10x | 0.17 |
| H₂O | | up to7 µl |
| **Total reaction volume** | - | 7 |

### Third stage of reaction:

| | **Stock concentration** | **Volume [µl]** |
|---|---|---|
| Mixture from stage 2 | | 7 |
| iPLEX buffer | 10x | 0.2 |
| iPLEX termination mixture | 10mM | 0.2 |
| Differentiating primer SEQ ID NO.47 | 7µM* | 0.94 -1.88* |
| iPLEX Pro polymerase | 32 U/µl | 0.041 |
| H₂O | | up to 9 µl |
| **Total reaction volume** | - | 9 |

| | | |
|---|---|---|
| * For differentiating primer concentrations: Low mass primers (< 6500 Da): final concentration 0.73µM High-mass primers ( ≥ 6500 Da): 1.46 µM | | |

The starting material for carrying out the method according to the invention is DNA that has been isolated from cells and tissues, or from a single cell. This DNA is subjected to prior digestion with the restriction enzyme Msel and preamplification. In the next step, DNA from a single cell is amplified in an optimized PCR reaction (Table 3, first step of the reaction; 2 minutes at 95°C, followed by 45 cycles: 30 seconds at 95°C; 30 seconds at 56°C; 60 seconds at 72°C, final step 5 minutes at 72°C) using the designed Primer 1 and Primer 2. Then (Table 3, second step of the reaction), the PCR products obtained, with lengths of 98-106 nucleotides (depending on the polymorphism studied), are treated with 0.5 U of SAP (shrimp alkaline phosphatase) to neutralize unused dNTPs. This involves incubation at 37 °C for 45 minutes, followed by enzyme inactivation at 85 °C for 5 minutes. This ensures that the dNTPs do not interfere with mass spectrometry reading in the next step. The next step (Table 3, third step of the reaction) is the iPLEX reaction, which uses differentiating primers that enable the detection of specific mutations. After purification, the diluted products (35 µl) are treated with 15 mg of ion-exchange resin (centrifuged for 15 minutes at 3200xg), then the products are applied onto a special analysis plate (SpectroCHIP) and subjected to a laser pulse that excites ions (a UV light pulse with a wavelength of 337 nm). The entire analysis is performed under vacuum conditions. The iPLEX reaction, products which differ by single nucleotides, are detected in the mass spectrometer, and the basis for variant identification is the ratio of their mass to charge (MALDI-TOF analysis), displayed as an easy-to-interpret graph (Fig. 1). The obtained result shows whether the wild-type allele or the mutant allele was present in the analyzed cell.

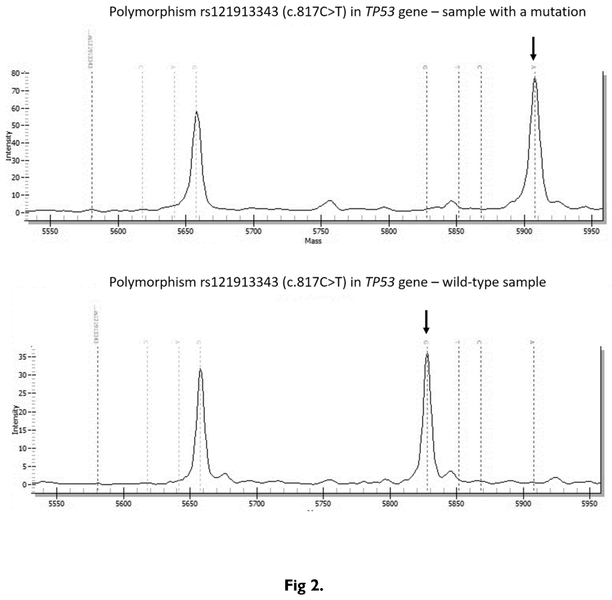

MALDI-TOF mass spectra analysis allows for a precise identification of variants present in the tested sample. By comparing the actual masses of the reaction products with the predicted masses, it is possible to determine which alleles are present in the sample. This allows the genotype of the studied locus to be identified. The analysis of results based on the mass spectra is composed of several stages:
1. Comparison of the mass spectra of the attached oligonucleotides with the predicted masses of those oligonucleotides. Each oligonucleotide detecting a specific allele (containing the attached nucleotide A, T, C, or G) has a defined mass, which was determined during the primer design.
2. Identification of alleles present in the sample based on comparing the actual masses of the obtained reaction products (oligonucleotides containing the attached nucleotide A, T, C, or G depending on the sample genotype) with the predicted masses of those oligonucleotides. This process allows precise identification of the variants (alleles) present in the sample.
3. Interpretation of the results, which involves assessing the sample genotype based on the detected alleles (Fig. 2):
   - If the mass spectrum contains a peak corresponding to the predicted mass of a given allele, it means that this allele is present in the sample.
   - The absence of a signal corresponding to the predicted mass of a given allele means that this allele is not present in the sample.
   - The presence of peaks corresponding to the masses of more than one allele indicates heterozygosity of the studied locus containing both alleles (e.g., wild-type allele and mutant allele).

The presence of a mutated allele indicates the cancerous nature of the sample, especially if such a mutation was not detected in the analyzed non-cancerous cells from the same individual (excluding germline mutation).

The described invention can be used as a technique for screening the cancer origin of the tested cells (it answers the question whether the cell is a cancerous cell or not).

The described invention may be used to analyze the heterogeneity of cancer. It is known that the mutational profile in cancer cells can differ between cells in the primary tumor. However, it is unknown which of the cancer cell populations within the tumor has the greatest potential to spread throughout the body (form distant metastases). Identifying a circulating cancer cell with a genotype (mutation status) different from the primary tumor (e.g., the absence of a mutation in the tumor but its presence in CTCs) indicates disease heterogeneity and the presence of metastatic cancer cells in the body that were not identified in the primary tumor. This may indicate the presence of another tumor site developing in the body.

The described invention may also be used to determine therapeutic targets and the possibility of selecting more effective treatment, e.g. in patients with mutations in the PIK3CA gene (including the mutations tested in the gene panel according to the present invention - rs121913273 and rs104886003), treatment in combination with alpelisib (clinical trial SOLAR-1 33246021 (André, et al., 2021) (Juric, et al., 2018)) resulted in extended overall survival of patients with metastases. Detection of these mutations in CTCs could therefore provide information about a possible treatment path.

The advantages of the described single-cell mutation analysis are:
- higher throughput than the Sanger sequencing method
- relatively simple and quick analysis
- high sensitivity (higher than in the Sanger method) - the ability to distinguish the mutant allele occurring at a low frequency (below 20%)
- Easy analysis of results, without the need for bioinformatical analysis. Products formed in the iPLEX reaction, differing by single nucleotides, are determined in a spectrometer, and the basis for variant identification is their mass-to-charge ratio (an example result generated during sample analysis is shown in Fig. 2).

### Sequence Listing

SEQUENCE LISTING

<110> University of Gdańsk
Medical University of Gdańsk

<120> Set of primers for detecting oncogenic mutations in DNA, method for detecting oncogenic
mutations in DNA and its use in assessing the cancer origin of cells

<130> 60276

<160> 69

<170> BiSSAP 1.3.6

<210> 1
<211> 30
<212> DNA
<213> Artificial Sequence

<220>
<223> nucleotide sequence of the forward primer for detecting polymorphism in the TP53 gene

<400> 1
acgttggatg agattctctt cctctgtgcg 30

<210> 2
<211> 30
<212> DNA
<213> Artificial Sequence

<220>
<223> Nucleotide sequence of the forward primer for detecting polymorphisms in the TP53 gene

<400> 2

acgttggatg ccaccatcca ctacaactac 30

<210> 3
<211> 30
<212> DNA
<213> Artificial Sequence

<220>
<223> Nucleotide sequence of the forward primer for detecting polymorphism in the TP53 gene

<400> 3
acgttggatg tccagtgtga tgatggtgag 30
<210> 4
<211> 30
<212> DNA
<213> Artificial Sequence

<220>
<223> Nucleotide sequence of the forward primer for detecting polymorphism in the TP53 gene

<400> 4
acgttggatg agattctctt cctctgtgcg 30

<210> 5
<211> 30
<212> DNA
<213> Artificial Sequence

<220>
<223> rs397516436F

<400> 5
acgttggatg agttgcaaac cagacctcag 30

<210> 6
<211> 29
<212> DNA
<213> Artificial Sequence

<220>
<223> rs28934578F

<400> 6
acgttggatg tgctcaccat cgctatctg                                29

<210> 7
<211> 30
<212> DNA
<213> Artificial Sequence

<220>
<223> rs121912666F

<400> 7
acgttggatg agttgcaaac cagacctcag                               30

<210> 8
<211> 30
<212> DNA
<213> Artificial Sequence

<220>
<223> Nucleotide sequence of the forward primer for detecting polymorphism in the PIK3CA
gene

<400> 8
acgttggatg aactgagcaa gaggctttgg                               30

<210> 9
<211> 30
<212> DNA
<213> Artificial Sequence

<220>
<223> Nucleotide sequence of the forward primer for detecting polymorphism in the PIK3CA
gene

<400> 9
acgttggatg tagcacttac ctgtgactcc                               30

<210> 10
<211> 30

<212> DNA
<213> Artificial Sequence

<220>
<223> Nucleotide sequence of the forward primer for detecting polymorphism in the PIK3CA
gene

<400> 10
acgttggatg tagcacttac ctgtgactcc                                        30

<210> 11
<211> 30
<212> DNA
<213> Artificial Sequence

<220>
<223> rs121913284F

<400> 11
acgttggatg gggttataaa tagtgcactc                                        30

<210> 12
<211> 30
<212> DNA
<213> Artificial Sequence

<220>
<223> rs121913277F

<400> 12
acgttggatg aactgagcaa gaggctttgg                                        30

<210> 13
<211> 30
<212> DNA
<213> Artificial Sequence

<220>
<223> rs121913274F

<400> 13

acgttggatg tagcacttac ctgtgactcc 30

<210> 14
<211> 30
<212> DNA
<213> Artificial Sequence

<220>
<223> rs121913286F

<400> 14
acgttggatg tacacgagat cctctctctg 30

<210> 15
<211> 30
<212> DNA
<213> Artificial Sequence

<220>
<223> rs1057519816F

<400> 15
acgttggatg tcctgatctc cttagacaac 30

<210> 16
<211> 29
<212> DNA
<213> Artificial Sequence

<220>
<223> rs121913471F

<400> 16
acgttggatg cgtggatgtc aggcagatg 29

<210> 17
<211> 30
<212> DNA
<213> Artificial Sequence

<220>
<223> rs121913470F

<400> 17
acgttggatg gggcttacgt ctaagatttc 30

<210> 18
<211> 30
<212> DNA
<213> Artificial Sequence

<220>
<223> rs796065354F

<400> 18
acgttggatg aagcccgctc atgatcaaac 30

<210> 19
<211> 30
<212> DNA
<213> Artificial Sequence

<220>
<223> rs1057519717F

<400> 19
acgttggatg cagcatgaag tgcaagaacg 30

<210> 20
<211> 30
<212> DNA
<213> Artificial Sequence
<220>
<223> rs2152506534F

<400> 20
acgttggatg cagcatgaag tgcaagaacg 30

<210> 21
<211> 30
<212> DNA
<213> Artificial Sequence

<220>
<223> rs2152506541F

<400> 21
acgttggatg cagcatgaag tgcaagaacg 30

<210> 22
<211> 29
<212> DNA
<213> Artificial Sequence

<220>
<223> rs1962456814F

<400> 22
acgttggatg ccggttccat ctacctttc 29

<210> 23
<211> 29
<212> DNA
<213> Artificial Sequence

<220>
<223> rs121434592F

<400> 23
acgttggatg tctgacgggt agagtgtgc 29

<210> 24
<211> 30
<212> DNA
<213> Artificial Sequence

<220>
<223> Nucleotide sequence of the reverse primer for detecting polymorphism in the TP53 gene

<400> 24
acgttggatg agtggtaatc tactgggacg 30

<210> 25
<211> 30
<212> DNA
<213> Artificial Sequence

<220>
<223> Nucleotide sequence of the reverse primer for detecting polymorphism in the TP53 gene

<400> 25
acgttggatg tccagtgtga tgatggtgag                                30

<210> 26
<211> 30
<212> DNA
<213> Artificial Sequence

<220>
<223> Nucleotide sequence of the reverse primer for detecting polymorphism in the TP53 gene

<400> 26
acgttggatg ccaccatcca ctacaactac                                30

<210> 27
<211> 30
<212> DNA
<213> Artificial Sequence

<220>
<223> Nucleotide sequence of the reverse primer for detecting polymorphism in the TP53 gene

<400> 27
acgttggatg agtggtaatc tactgggacg                                30

<210> 28
<211> 30
<212> DNA
<213> Artificial Sequence

<220>
<223> rs397516436R
<400> 28

acgttggatg gtgtggagta tttggatgac 30

<210> 29
<211> 30
<212> DNA
<213> Artificial Sequence

<220>
<223> rs28934578R

<400> 29
acgttggatg ccatctacaa gcagtcacag 30

<210> 30
<211> 30
<212> DNA
<213> Artificial Sequence

<220>
<223> rs121912666R

<400> 30
acgttggatg gtgtggagta tttggatgac 30

<210> 31
<211> 30
<212> DNA
<213> Artificial Sequence

<220>
<223> Nucleotide sequence of the reverse primer for detecting polymorphism in the PIK3CA
gene

<400> 31
acgttggatg tccatttttg ttgtccagcc 30

<210> 32
<211> 30
<212> DNA
<213> Artificial Sequence

<220>
<223> Nucleotide sequence of the reverse primer for detecting polymorphism in the PIK3CA
gene

<400> 32
acgttggatg gcaatttcta cacgagatcc                                30

<210> 33
<211> 30
<212> DNA
<213> Artificial Sequence

<220>
<223> Nucleotide sequence of the reverse primer for amplification of the polymorphism in the
PIK3CA gene

<400> 33
acgttggatg tacacgagat cctctctctg                                30

<210> 34
<211> 30
<212> DNA
<213> Artificial Sequence

<220>
<223> rs121913284R

<400> 34
acgttggatg catcagcatt tgactttacc                                30

<210> 35
<211> 29
<212> DNA
<213> Artificial Sequence

<220>
<223> rs121913277R

<400> 35
acgttggatg ttgtgtggaa gatccaatc                                29

<210> 36
<211> 30
<212> DNA
<213> Artificial Sequence

<220>
<223> rs121913274R

<400> 36
acgttggatg tacacgagat cctctctctg                                30
<210> 37
<211> 30
<212> DNA
<213> Artificial Sequence

<220>
<223> rs121913286R

<400> 37
acgttggatg tagcacttac ctgtgactcc                                30

<210> 38
<211> 29
<212> DNA
<213> Artificial Sequence

<220>
<223> rs1057519816R

<400> 38
acgttggatg tcacctcttg gttgtgcag                                29

<210> 39
<211> 29
<212> DNA
<213> Artificial Sequence

<220>
<223> rs121913471R

<400> 39

acgttggatg ccaggaagca tacgtgatg 29

<210> 40
<211> 29
<212> DNA
<213> Artificial Sequence

<220>
<223> rs121913470R

<400> 40
acgttggatg gaaaattcca gtggccatc 29

<210> 41
<211> 30
<212> DNA
<213> Artificial Sequence

<220>
<223> rs796065354R

<400> 41
acgttggatg aacaaggcac tgaccatctg 30

<210> 42
<211> 29
<212> DNA
<213> Artificial Sequence

<220>
<223> rs1057519717R

<400> 42
acgttggatg gctagtgggc gcatgtagg 29

<210> 43
<211> 29
<212> DNA
<213> Artificial Sequence

<220>
<223> rs2152506534R

<400> 43
acgttggatg gctagtgggc gcatgtagg 29

<210> 44
<211> 29
<212> DNA
<213> Artificial Sequence

<220>
<223> rs2152506541R

<400> 44
acgttggatg gctagtgggc gcatgtagg 29

<210> 45
<211> 30
<212> DNA
<213> Artificial Sequence

<220>
<223> rs1962456814R

<400> 45
acgttggatg tgtagctctc ggcgtcaaag 30

<210> 46
<211> 30
<212> DNA
<213> Artificial Sequence

<220>
<223> rs121434592R

<400> 46
acgttggatg ttcttgagga ggaagtagcg 30

<210> 47
<211> 17

<212> DNA
<213> Artificial Sequence

<220>
<223> Differentiating primer sequence for detecting polymorphism in the TP53 gene

<400> 47
ggacagcttt gaggtgc 17

<210> 48
<211> 16
<212> DNA
<213> Artificial Sequence

<220>
<223> Differentiating primer sequence for detecting polymorphism in the TP53 gene

<400> 48
ttgaggatgg gcctcc 16

<210> 49
<211> 15
<212> DNA
<213> Artificial Sequence

<220>
<223> Nucleotide sequence of the differentiating primer for detecting polymorphism in the TP53
gene

<400> 49
tgggcggcat gaacc 15

<210> 50
<211> 18
<212> DNA
<213> Artificial Sequence

<220>
<223> Nucleotide sequence of the differentiating primer for detecting polymorphism in the TP53
gene

<400> 50
cggaacagct ttgaggtg 18

<210> 51
<211> 17
<212> DNA
<213> Artificial Sequence

<220>
<223> rs397516436D

<400> 51
atgacagaaa cactttt 17

<210> 52
<211> 15
<212> DNA
<213> Artificial Sequence

<220>
<223> rs28934578D

<400> 52
cggaggttgt gaggc 15

<210> 53
<211> 18
<212> DNA
<213> Artificial Sequence
<220>
<223> rs121912666D

<400> 53
atagtgtggt ggtgccct 18

<210> 54
<211> 15
<212> DNA
<213> Artificial Sequence

<220>

<223> Nucleotide sequence of the differentiating primer for detecting polymorphism in the
PIK3CA gene

<400> 54
gtccagccac catga                                                    15

<210> 55
<211> 17
<212> DNA
<213> Artificial Sequence

<220>
<223> Nucleotide sequence of the differentiating primer for detecting polymorphism in the
PIK3CA gene

<400> 55
cacgagatcc tctctct                                                  17

<210> 56
<211> 18
<212> DNA
<213> Artificial Sequence

<220>
<223> Nucleotide sequence of the differentiating primer for detecting polymorphism in the
PIK3CA gene

<400> 56
cctctctctg aaatcact                                                 18

<210> 57
<211> 25
<212> DNA
<213> Artificial Sequence

<220>
<223> rs121913284D

<400> 57
ccttatcaat gtctcgaata tttac                                         25

<210> 58
<211> 19
<212> DNA
<213> Artificial Sequence

<220>
<223> rs121913277D

<400> 58
atttttgttg tccagccac 19

<210> 59
<211> 17
<212> DNA
<213> Artificial Sequence

<220>
<223> rs121913274D

<400> 59
tctctctgaa atcactg 17

<210> 60
<211> 16
<212> DNA
<213> Artificial Sequence

<220>
<223> rs121913286D

<400> 60
agaaaatctt tctcct 16

<210> 61
<211> 18
<212> DNA
<213> Artificial Sequence

<220>
<223> rs1057519816D
<400> 61

gggcagacga gggtgcag 18

<210> 62
<211> 20
<212> DNA
<213> Artificial Sequence

<220>
<223> rs121913471D

<400> 62
aagcatacgt gatggctggt 20

<210> 63
<211> 16
<212> DNA
<213> Artificial Sequence

<220>
<223> rs121913470D

<400> 63
gtggccatca aagtgt 16

<210> 64
<211> 19
<212> DNA
<213> Artificial Sequence

<220>
<223> rs796065354D

<400> 64
ggacaaggcc aggctgttc 19

<210> 65
<211> 22
<212> DNA
<213> Artificial Sequence

<220>
<223> rs1057519717D

<400> 65
catctccagc agcaggtcat ag 22

<210> 66
<211> 15
<212> DNA
<213> Artificial Sequence

<220>
<223> rs2152506534D

<400> 66
ccagcagcag gtcat 15

<210> 67
<211> 18
<212> DNA
<213> Artificial Sequence

<220>
<223> rs2152506541D

<400> 67
atctccagca gcaggtca 18

<210> 68
<211> 22
<212> DNA
<213> Artificial Sequence

<220>
<223> rs1962456814D

<400> 68
gggtggcagg gctccggctc ct 22

<210> 69
<211> 24

<212> DNA
<213> Artificial Sequence

<220>
<223> rs121434592D

<400> 69
gtggccgcca ggtcttgatg tact 24

### List of publications:

1. André, F., E. M. Ciruelos, D. Juric, S. Loibl, M. Campone, I. A. Mayer, G. Rubovszky, T. Yamashita, B. Kaufman, Y. S. Lu, K. Inoue, Z. Pápai, M. Takahashi, F. Ghaznawi, D. Mills, M. Kaper, M. Miller, P. F. Conte, H. Iwata and H. S. Rugo. (2021), 'Alpelisib Plus Fulvestrant for Pik3ca-Mutated, Hormone Receptor-Positive, Human Epidermal Growth Factor Receptor-2-Negative Advanced Breast Cancer: Final Overall Survival Results from Solar-1', Ann Oncol Vol. 32, No. 2, pp. 208-217.
2. Borgström, E., M. Paterlini, J. E. Mold, J. Frisen and J. Lundeberg. (2017), 'Comparison of Whole Genome Amplification Techniques for Human Single Cell Exome Sequencing', PLoS One Vol. 12, No. 2, pp. e0171566.
3. Juric, D., J. Rodon, J. Tabernero, F. Janku, H. A. Burris, J. H. M. Schellens, M. R. Middleton, J. Berlin, M. Schuler, M. Gil-Martin, H. S. Rugo, R. Seggewiss-Bernhardt, A. Huang, D. Bootle, D. Demanse, L. Blumenstein, C. Coughlin, C. Quadt and J. Baselga. (2018), 'Phosphatidylinositol 3-Kinase A -Selective Inhibition with Alpelisib (Byl719) in Pik3ca-Altered Solid Tumors: Results from the First-in-Human Study', J Clin Oncol Vol. 36, No. 13, pp. 1291-1299.
4. Khoo, B. L., M. E. Warkiani, D. S. Tan, A. A. Bhagat, D. Irwin, D. P. Lau, A. S. Lim, K. H. Lim, S. S. Krisna, W. T. Lim, Y. S. Yap, S. C. Lee, R. A. Soo, J. Han and C. T. Lim. (2014), ' Clinical Validation of an Ultra High-Throughput Spiral Microfluidics for the Detection and Enrichment of Viable Circulating Tumor Cells ', PLoS One Vol. 9, No. 7, pp. e99409.
5. Peeters, D. J., B. De Laere, G. G. Van den Eynden, S. J. Van Laere, F. Rothé, M. Ignatiadis, A. M. Sieuwerts, D. Lambrechts, A. Rutten, P. A. van Dam, P. Pauwels, M. Peeters, P. B. Vermeulen and L. Y. Dirix. (2013), 'Semiautomated Isolation and Molecular Characterization of Single or Highly Purified Tumor Cells from Cellsearch Enriched Blood Samples Using Dielectrophoretic Cell Sorting', Br J Cancer Vol. 108, No. 6, pp. 1358-67.
6. Topa, J., P. Grešner, A. J. Zaczek and A. Markiewicz. (2022), 'Breast Cancer Circulating Tumor Cells with Mesenchymal Features-an Unreachable Target?', Cell Mol Life Sci Vol. 79, No. 2, pp. 81.

## Claims

1. A set of primers for the detection of oncogenic mutations, at the level of single circulating tumor cells, in the *TP53, PIK3CA, ERBB2, ESR1, CDH1* and *AKT1* genes **characterized in that** the forward primer for amplifying the polymorphism in the *TP53* gene is selected from SEQ ID NO.1 to SEQ ID NO.7, the reverse primer is selected from the group SEQ ID NO.24 to SEQ ID NO.30, the forward primer for amplifying the polymorphism in the *PIK3CA* gene is selected from SEQ ID NO.8 to SEQ ID NO.14, and the reverse primer is selected from the group SEQ ID NO.31 to SEQ ID NO.37, the forward primer for amplifying the polymorphism in the *ERBB2* gene is selected from SEQ ID NO.15 to SEQ ID NO.17, and the reverse primer is selected from the group SEQ ID NO.38 to SEQ ID NO.40, the forward primer for amplifying the polymorphism in the *ESR1* gene is selected from SEQ ID NO.18 to SEQ ID NO.21, and the reverse primer is selected from the group SEQ ID NO.41 to SEQ ID NO.44, the forward primer for the amplification of the polymorphism in the *CDH1* gene is SEQ ID NO.22 and the reverse primer is SEQ ID NO.45, while the forward primer for the amplification of the polymorphism in the *AKT1* gene is SEQ ID NO.23 and the reverse primer is SEQ ID NO.46, wherein the kit comprises a differentiating primer which for the *TP53* gene is selected from the group of SEQ ID NO.47 to SEQ ID NO.53, for the *PIK3CA* gene is selected from the group of SEQ ID NO.54 to SEQ ID NO.60, for the *ERBB2* gene is selected from the group of SEQ ID NO.61 to SEQ ID NO.63, for the *ESR1* gene is selected from the group of SEQ ID NO.64 to SEQ ID NO.67, for the *CDH1* gene is SEQ ID NO.68, and for the *AKT1* gene is SEQ ID NO.69.

2. The set of primers according to claim 1, **characterized in that** when the forward primer has the sequence SEQ ID NO. 1, the reverse primer has the sequence SEQ ID NO. 24 and the differentiating primer has the sequence SEQ ID NO. 47 or
when the forward primer has the sequence SEQ ID NO.2, the reverse primer has the sequence SEQ ID NO.25 and the differentiating primer has the sequence SEQ ID NO.48 or
when the forward primer has the sequence SEQ ID NO.3, the reverse primer has the sequence SEQ ID NO.26 and the differentiating primer has the sequence SEQ ID NO.49 or
when the forward primer has the sequence SEQ ID NO.4, the reverse primer has the sequence SEQ ID NO.27 and the differentiating primer has the sequence SEQ ID NO.50 or
when the forward primer has the sequence SEQ ID NO.5, the reverse primer has the sequence SEQ ID NO.28 and the differentiating primer has the sequence SEQ ID NO.51 or
when the forward primer has the sequence SEQ ID NO.6, the reverse primer has the sequence SEQ ID NO.29 and the differentiating primer has the sequence SEQ ID NO.52 or
when the forward primer has the sequence SEQ ID NO.7, the reverse primer has the sequence SEQ ID NO.30 and the differentiating primer has the sequence SEQ ID NO.53 or
when the forward primer has the sequence SEQ ID NO.8, the reverse primer has the sequence SEQ ID NO.31 and the differentiating primer has the sequence SEQ ID NO.54 or
when the forward primer has the sequence SEQ ID NO.9, the reverse primer has the sequence SEQ ID NO.32 and the differentiating primer has the sequence SEQ ID NO.55 or
when the forward primer has the sequence SEQ ID NO.10, the reverse primer has the sequence SEQ ID NO.33 and the differentiating primer has the sequence SEQ ID NO.56 or
when the forward primer has the sequence SEQ ID NO.11, the reverse primer has the sequence SEQ ID NO.34 and the differentiating primer has the sequence SEQ ID NO.57 or
when the forward primer has the sequence SEQ ID NO.12, the reverse primer has the sequence SEQ ID NO.35 and the differentiating primer has the sequence SEQ ID NO.58 or
when the forward primer has the sequence SEQ ID NO.13, the reverse primer has the sequence SEQ ID NO.36 and the differentiating primer has the sequence SEQ ID NO.59 or
when the forward primer has the sequence SEQ ID NO.14, the reverse primer has the sequence SEQ ID NO.37 and the differentiating primer has the sequence SEQ ID NO.60 or
when the forward primer has the sequence SEQ ID NO.15, the reverse primer has the sequence SEQ ID NO.38 and the differentiating primer has the sequence SEQ ID NO.61 or
when the forward primer has the sequence SEQ ID NO.16, the reverse primer has the sequence SEQ ID NO.39 and the differentiating primer has the sequence SEQ ID NO.62 or
when the forward primer has the sequence SEQ ID NO.17, the reverse primer has the sequence SEQ ID NO.40 and the differentiating primer has the sequence SEQ ID NO.63 or
when the forward primer has the sequence SEQ ID NO.18, the reverse primer has the sequence SEQ ID NO.41 and the differentiating primer has the sequence SEQ ID NO.64 or
when the forward primer has the sequence SEQ ID NO.19, the reverse primer has the sequence SEQ ID NO.42 and the differentiating primer has the sequence SEQ ID NO.65 or
when the forward primer has the sequence SEQ ID NO.20, the reverse primer has the sequence SEQ ID NO.43 and the differentiating primer has the sequence SEQ ID NO.66 or
when the forward primer has the sequence SEQ ID NO.21, the reverse primer has the sequence SEQ ID NO.44 and the differentiating primer has the sequence SEQ ID NO.67 or
when the forward primer has the sequence SEQ ID NO.22, the reverse primer has the sequence SEQ ID NO.45 and the differentiating primer has the sequence SEQ ID NO.68 or
when the forward primer has the sequence SEQ ID NO.23, the reverse primer has the sequence SEQ ID NO.46 and the differentiating primer has the sequence SEQ ID NO.69.

3. The set of primers according to claim 1 or 2, **characterized in that** the sequence of each primer contains a constant sequence ACGTTGGATG necessary for detecting the amplified DNA in a mass spectrometer.

4. A method for detecting oncogenic mutations at the level of single cancer cells in the *TP53, PIK3CA, ERBB2, ESR1, CDH1* and *AKT1* genes using mass spectrometry technology, comprising the steps of cell lysis and DNA digestion with the Msel restriction enzyme, amplification of DNA from a single cell, preferably using the commercial Ampli1^{™} WGA kit and amplifying genome fragments containing selected oncogenic mutations, **characterized in that** the genome fragments containing the oncogenic mutation, comprising sequences between the Msel restriction enzyme cleavage sites in step a) are amplified using a set of primers comprising a forward primer and a reverse primer as disclosed in claim **1,** in step b) the product obtained in step a) is treated with alkaline phosphatase, in step c) the product from step b) is subjected to iPLEX amplification with a differentiating primer as disclosed in claim 1 to detect a specific mutation, then in step d) the product from step c) is purified on an ion exchange resin, and then in step e) MALDI-TOF analysis of the iPLEX reaction products is performed in a spectrometer and their mass spectra are compared, and in step f) the genotype of the sample is assessed on the basis of the detected alleles and the results are interpreted.

5. The method according to claim 4, **characterized in that** the forward primer for amplifying the polymorphism in the *TP53* gene in step a) is selected from SEQ ID NO. 1 to SEQ ID NO. 7, and the reverse primer is selected from SEQ ID NO. 24 to SEQ ID NO. 30.

6. The method according to claim 4, **characterized in that** the forward primer for amplifying the polymorphism in the *PIK3CA* gene in step a) is selected from SEQ ID NO. 8 to SEQ ID NO. 14, and the reverse primer is selected from SEQ ID NO. 31 to SEQ ID NO. 37.

7. The method according to claim 4, **characterized in that** the forward primer for amplifying the polymorphism in the *ERBB2* gene in step a) is selected from SEQ ID NO. 15 to SEQ ID NO. 17, and the reverse primer is selected from SEQ ID NO. 38 to SEQ ID NO. 40.

8. The method according to claim 4, **characterized in that** the forward primer for amplifying the polymorphism in the *ESR1* gene in step a) is selected from SEQ ID NO.18 to SEQ ID NO.21, and the reverse primer is selected from SEQ ID NO.41 to SEQ ID NO.44.

9. The method according to claim 4, **characterized in that** the forward primer for amplifying the polymorphism in the *CDH1* gene in step a) is SEQ ID NO.22 and the reverse primer is selected from SEQ ID NO.45.

10. The method according to claim 4, **characterized in that** the forward primer for amplifying the polymorphism in the *AKT1* gene in step a) is SEQ ID NO.23 and the reverse primer is SEQ ID NO.46.

11. The method according to any one of claims 4 to 10, **characterized in that** the amplification in step a) takes place in a thermal cycler according to the scheme: 2 minutes at 95 °C, then 45 cycles of 30 seconds at 95 °C, 30 seconds at 56 °C, 60 seconds at 72 °C and includes a final step of 5 minutes at 72 °C.

12. The method according to any one of claims 4 to 11, **characterized in that** in step b) the incubation with alkaline phosphatase is carried out at a temperature of 36-38 °C for at least 40 minutes, preferably at a temperature of 37 °C for 45 minutes.

13. The method according to any one of claims 4 to 12, **characterized in that** the iPLEX reaction products of step c) are purified in step d) with 15 mg of ion exchange resin by centrifugation for 15 minutes at 3200 x g.

14. The method according to any one of claims 4 to 13, **characterized in that** in step e) the products are applied to an analysis plate and subjected to a UV laser pulse with a wavelength of 337 nm exciting the ions, preferably the analysis is carried out in a vacuum, preferably on a SpectroCHIP analysis plate.

15. Use of the method according to any one of claims 4 to 14 for assessing the tumor origin of cells or for analyzing the heterogeneity of a tumor disease and the presence of metastatic cells in the body.
